# EUROPÄISCHE PATENTSCHRIFT

(11) **EP 3 664 860 B1**
(45) Veröffentlichungstag und Bekanntmachung des Hinweises auf die Patenterteilung: **01.05.2024**
(21) Anmeldenummer: 18753175.1
(22) Anmeldetag: 10.08.2018
(51) Int. Cl.: A61M 3/02, A61M 37/00, A61M 5/152, A61M 5/28, A61F 9/00, B65D 75/36, A61J 1/06

(54) **VERABREICHUNGSVORRICHTUNG UND VERFAHREN ZUR HERSTELLUNG EINER SOLCHEN**
ADMINISTRATION DEVICE AND METHOD FOR PRODUCING SAME
DISPOSITIF D'ADMINISTRATION ET PROCÉDÉ POUR SA PRODUCTION

(30) Priorität: 11.08.2017 CH 10162017
(43) Veröffentlichungstag der Anmeldung: 17.06.2020
(73) Patentinhaber: Pantec AG, 6362 Stansstad (CH)
(72) Erfinder: ROHRER, Hans-Peter, 4313 Möhlin (CH); NICK, Jürgen, 79395 Neuenburg (CH)
(74) Vertreter: Latscha Schöllhorn Partner AG
(86) Internationale Anmeldenummer: PCT/EP2018/071815
(87) Internationale Veröffentlichungsnummer: WO 2019/030401

(56) Entgegenhaltungen:
- DE-A1- 3 122 237
- DE-A1- 10 009 627
- US-A1- 2006 131 189
- US-A1- 2014 346 071

## Beschreibung

### Technisches Gebiet

Die Erfindung betrifft eine Verabreichungsvorrichtung gemäss dem Oberbegriff des unabhängigen Anspruchs 1 sowie ein Verfahren zu deren Herstellung. Solche Verabreichungsvorrichtungen umfassen typischweise einen Grundkörper, der eine Auslassöffnung und eine Kammer zur Aufnahme einer zu verabreichenden fliessfähigen Substanz aufweist, der aus einem ersten Folienabschnitt und einem mit dem ersten Folienabschnitt verbundenen zweiten Folienabschnitt hergestellt ist, und der dazu ausgestaltet ist, dass die Substanz mittels Zusammendrücken der Kammer durch die Auslassöffnung aus der Verabreichungsvorrichtung austragbar beziehungsweise ausbringbar ist.

### Stand der Technik

Zu Pflegezwecken und zur therapeutischen Behandlung verschiedener Krankheiten werden heutzutage fliessfähige beziehungsweise flüssige Substanzen eingesetzt, die in unterschiedlicher Form verabreicht werden. Beispielsweise werden solche Substanzen in Körperöffnungen wie unter anderem Mund oder Ohren abgegeben oder in den Körper eines Patienten injiziert. Dazu wird die Substanz üblicherweise mittels einer spezifischen Verabreichungsvorrichtung angewendet, die eine gezielte bestimmungsgemässe Applikation ermöglicht.

Beispielsweise werden zur Behandlung und Pflege der Augen häufig Pflegeprodukte und Medikamente in Form von Augentröpfchen in die Augen verabreicht. Dazu ist es bekannt, die Pflegeprodukte beziehungsweise Medikamente als flüssige Substanz portionenweise in Kunststoffbehältern bereit zu stellen. Zur einfachen Anwendung weisen solche Kunststoffbehälter häufig eine schnabelförmige Auslassöffnung auf. In der Anwendung wird die Auslassöffnung freigelegt und am Auge positioniert. Danach wird die flüssige Substanz tröpfchenweise durch ein Zusammendrücken des Behälters aus der Auslassöffnung ausgebracht und in das Auge abgegeben.

In ähnlicher Art werden auch Medikamente angeboten, die zur oralen Verabreichungen vorgesehen sind. Beispielsweise werden Schluckimpfungen mittels ähnlicher Verabreichungsvorrichtungen angewendet.

Auch ist es bekannt, zur subkutanen, intravenösen oder intradermalen Injektion vorgesehene Substanzen portioniert in einer Verabreichungsvorrichtung bereit zu stellen. Klassischerweise werden dazu befüllte Einwegspritzen eingesetzt. Alternativ dazu ist beispielsweise in der EP 1 092 444 B1 eine intradermale Verabreichungsvorrichtung beschrieben, die einen Grundkörper aus zwei Lagen eines thermoplastischen Materials umfasst. Die zwei Lagen bilden ein Reservoir, in dem eine pharmazeutische Substanz angeordnet ist, und eine mit dem Reservoir durchgängig verbundene Auslassöffnung. In die Auslassöffnung ist ein Verbindungsstück eingelassen, in das eine Nadeleinrichtung eingesetzt ist. Das Verbindungsstück weist eine Dichtmembran auf, welche die Auslassöffnung abdichtet. Die Nadeleinrichtung umfasst eine Nadel, die einerseits zur Injektion vorgesehen ist und andererseits die Dichtmembran des Verbindungsstücks durchsticht, wenn die Nadeleinrichtung in das Verbindungsstück eingesetzt wird. Bei eingesetzter Nadeleinrichtung kann die Substanz durch Zusammendrücken des Reservoirs durch die Auslassöffnung und die Nadeleinrichtung ausgebracht werden.

Eine weitere Verabreichungsvorrichtung ist aus US 2014/346071 bekannt.

Ein Nachteil bei den bekannten Verabreichungsvorrichtungen ist, dass die Art der Verabreichung üblicherweise vorgegeben ist. Das führt dazu, dass üblicherweise für jede Art der Verabreichung die Herstellung wie beispielsweise die dabei eingesetzten Werkzeuge angepasst werden muss. In gewissen Anwendungen kann es auch gewünscht sein, erst nach der Herstellung der Verabreichungsvorrichtung oder gar erst bei der Verabreichung selbst die beste Art zu wählen. In solchen Fällen müssen gleich mehrere alternative Verabreichungsvorrichtung bereit gestellt beziehungsweise verwendet werden. Bei den erwähnten zur Injektion vorgesehenen alternativen Verabreichungsvorrichtungen kann zudem das Einsetzen der Nadeleinrichtung problematisch sein. Beispielsweise besteht beim Einsetzen der Nadeleinrichtung ein Risiko, dass ein Teil der Substanz unmittelbar austritt, sodass die genau Dosis nicht mehr gewährleistet werden kann. Auch kann beim Durchstechen der Dichtmembran eine Kontamination der zu verabreichenden Substanz auftreten. Auch kann die Herstellung mit dem Verbindungsstück und seiner Dichtmembran verhältnismässig aufwendig sein.

Der vorliegenden Erfindung liegt daher die Aufgabe zu Grunde, eine Vorrichtung vorzuschlagen, die eine präzise, hygienische und kostengünstige Verabreichung von Medikamenten oder anderen sterilen Substanzen auf unterschiedliche Weise ermöglicht sowie ein Verfahren zur effizienten und einfachen Herstellung einer solchen.

### Darstellung der Erfindung

Die Aufgabe wird erfindungsgemäss durch eine Verabreichungsvorrichtung gelöst, wie sie im unabhängigen Anspruch 1 definiert ist, und durch ein Herstellungsverfahren, wie es im unabhängigen Anspruch 8 definiert ist. Vorteilhafte Ausführungsvarianten der Erfindung ergeben sich aus den abhängigen Ansprüchen.

Das Wesen der Erfindung besteht im Folgenden: Eine Verabreichungsvorrichtung umfasst einen Grundkörper, der eine Auslassöffnung und eine Kammer zur Aufnahme einer zu verabreichenden fliessfähigen Substanz aufweist. Der Grundkörper ist aus einem ersten Folienabschnitt und einem mit dem ersten Folienabschnitt verbundenen zweiten Folienabschnitt hergestellt ist. Zudem ist der Grundkörper dazu ausgestaltet, dass die Substanz mittels Zusammendrücken der Kammer durch die Auslassöffnung austragbar ist.

Die Verabreichungsvorrichtung umfasst weiter einen dicht mit der Auslassöffnung verbundenen Ausgussadapter mit einem Anschluss. Die Verbindung des ersten Folienabschnitts mit dem zweiten Folienabschnitt dichtet die Kammer ab. Die Verbindung weist zudem mehrer Bereiche auf, die einen festeren Bereich und einen zwischen der Kammer und der Auslassöffnung angeordneten weniger festen Bereich umfassen. Der weniger feste Bereich der Verbindung ist dazu ausgebildet, sich beim Zusammendrücken der Kammer zum Austragen der Substanz durch die Auslassöffnung zu lösen. Der festere Bereich der Verbindung ist dazu ausgebildet, beim Zusammendrücken der Kammer zum Austragen der Substanz durch die Auslassöffnung verbunden zu bleiben. Die Substanz ist mittels Zusammendrücken der Kammer quasi ausschliesslich durch die Auslassöffnung und durch den Anschluss des Ausgussadapters austragbar beziehungsweise ausbringbar.

Unter dem Begriff "Verabreichungsvorrichtung" kann im Zusammenhang mit der Erfindung eine Vorrichtung verstanden werden, die zur Abgabe einer kontrollierten definierbaren Menge der Substanz in einer vorbestimmten Form ausgebildet ist. Sie kann insbesondere eine Portionenpackung sein, die für eine einzelne oder auch für wenige mehrere Anwendungen vorgesehen ist.

Die Substanz kann insbesondere ein Medikament wie beispielsweise ein Impfstoff oder ein Pflegemittel sein. Damit sie erfindungsgemäss verabreicht werden kann, liegt die Substanz vor Applikation in einer fliessfähigen Form vor. Dabei kann sie insbesondere flüssig aber auch gasförmig oder pulverförmig sein. Die Verabreichungsvorrichtung kann als Dispenser bezeichnet werden.

Der Grundkörper kann formstabil sein, wobei sich in diesem Zusammenhang der Begriff "formstabil" auf eine Ausgestaltung bezieht, die ohne äusseren Einfluss seine Form hält. Insbesondere können der Grundkörper und die Kammer während der Handhabung der Verabreichungsvorrichtung wie beispielsweise während der Lagerung, dem Verkauf, der Lieferung, der Entnahme bis zur eigentlichen Verabreichung eine feste Form haben. Erst zur Verabreichung kann der Grundkörper dann deformiert werden, wobei dazu die Kammer beispielsweise mit der Hand gedrückt und die Substanz ausgepresst wird.

Der Begriff "Folienabschnitt" kann sich im Zusammenhang mit dem Grundkörper auf eine ein- oder mehrteilige Struktur beziehen. Insbesondere können der erste Folienabschnitt und der zweite Folienabschnitt zwei unterschiedliche Teile und insbesondere zwei Folien sein, die miteinander verbunden sind. Oder der erste und der zweite Folienabschnitt können einteilig also insbesondere eine Folie sein. Dabei können sie beispielsweise über Falten zueinander positioniert und ausgerichtet und dann miteinander verbunden sein.

Die Folienabschnitte könne aus einem verhältnismässig reissfesten Kunststoff wie beispielsweise aus einem Polyvinylchlorid hergestellt sein. Alternativ dazu können die Folienabschnitte auch aus Aluminium beziehungsweise einem Aluminiumlaminat hergestellt sein. Grundsätzlich kann jede formbare Folie als Folienabschnitt verwendet werden. Das verwendete Material kann dabei gezielt auf die jeweiligen Anforderungen der Substanz beziehungsweise des Medikaments oder des Produkts wie beispielsweise Barrierewirkung, Lichtschutz oder ähnliche abgestimmt werden. Der Kunststoff der Folienabschnitte kann durchsichtig sein, sodass eine visuelle Prüfung des Inhalts der Kammer von aussen her möglich ist.

Die Verbindung der ersten und zweiten Folienabschnitte ist insbesondere dergestalt, dass die Kammer abgedichtet beziehungsweise hermetisch abgeriegelt ist. Dadurch kann die Substanz sicher und steril aufbewahrt sein. Der festere Bereich der Verbindung kann insbesondere diesbezüglich fest sein, dass er beim Zusammendrücken der Kammer hält und sich nicht oder zumindest nicht in grösserem Umfang löst. Er kann somit dauerhaft dicht sein. Wichtig ist dabei, dass der festere Bereich ausreichend fest ist, damit er beim Zusammendrücken der Kammer verbunden bleiben kann. Der weniger feste Bereich der Verbindung weist insbesondere im Vergleich zum festeren Bereich eine geringere Festigkeit auf. Insbesondere ist seine Festigkeit gering genug, dass er sich beim Zusammenpressen der Kammer ausreichend loslöst, damit die Substanz zur Ausgussöffnung durchdringen kann. Gleichzeitig ist seine Festigkeit gross genug, dass die Kammer dicht verschlossen ist.

Der Begriff "quasi ausschliesslich" kann sich im Zusammenhang mit dem Austragen der Substanz durch den Anschluss des Ausgussadapters darauf beziehen, dass im Wesentlichen keine Substanz verloren geht. Insbesondere kann die Verbindung dergestalt sein, dass sie in ihrem festen Bereich dicht bleibt und lediglich im weniger festen Bereich zwischen Kammer und Ausgangsöffnung gelöst und somit durchlässig ist.

Über ihren Ausgussadapter kann die erfindungsgemässe Verabreichungsvorrichtung spezifisch auf die vorgesehene Anwendung hin optimiert werden. Insbesondere kann ein passend auf die gewünschte Verabreichungsart der Substanz, wie beispielsweise Spritzen, Tropfen, Aufstreichen oder Injizieren, ein passendes Teil, wie beispielsweise eine Ausguss, ein Tröpfchenspender oder eine Injektionsnadel einer gewünschten Länge am Anschluss des Ausgussadapters montiert werden. Dadurch erlaubt die Verabreichungsvorrichtung eine präzise und hygienische werkzeuglose Verabreichung von Medikamenten oder anderen Substanzen in einer gewünschten Art und Weise. Die Verabreichungsvorrichtung kann manuell oder auch über eine (semi-)manuelle Vorrichtung appliziert werden

Die Verbindung der beiden Folienabschnitte und insbesondere ihre Ausgestaltung mit den unterschiedlich festen Bereichen ermöglicht es, dass eine Abdichtung der Kammer möglich ist, die gezielt aufbricht beziehungsweise geöffnet wird. Insbesondere ermöglicht sie, dass die Kammer erst dann geöffnet wird, wenn die Substanz appliziert wird. Dadurch kann die Sterilität der Substanz bis zum Anwendungszeitpunkt gesichert werden. Auch ermöglicht sie, dass auf zusätzlich Komponenten wie beispielsweise eine Nadel oder etwas ähnliches zum Öffnen der Kammer verzichtet werden kann. Dies kann vorteilhaft zur Verhinderung von Kontaminationen sein und eine vergleichseise einfache Anwendung ermöglichen. Weiter ermöglicht sie eine vergleichsweise einfache und effiziente Herstellung der Verabreichungsvorrichtung. Insbesondere kann sie in einem Verfahren hergestellt werden, wie es aus der Herstellung von herkömmlichen Blisterverpackungen her bekannt ist. Dadurch kann die Verabreichungsvorrichtung auch verhältnismässig kostengünstig produziert werden. Auch die Herstellung in einer sterilen Umgebung ist verhältnismässig einfach möglich, was insbesondere bei Substanzen für eine medizinische Anwendung vorteilhaft sein kann. Weiter ermöglicht die Verabreichungsvorrichtung eine hohe Flexibilität bei der Auswahl der eingesetzten Materialien. Dadurch können auf einfache Weise Materialien verwendet werden, die beispielsweise für die Substanz bevorzugte Barriereeigenschaften aufweisen oder die optisch bevorzugt sind.

Vorzugsweise ist der Ausgussadapter aus einem festen Material hergestellt, das bei einem Zusammendrücken der Kammer zum Austragen der Substanz im Wesentlichen seine Form beibehält. Dadurch kann der Ausgussadapter während der Verabreichung der Substanz für die Abgabe bevorzugt ausgebildet sein und bleiben, insbesondere auch während die Kammer zusammengedrückt wird. So kann gewährleistet werden, dass keine Beeinträchtigung des Anschlusses des passenden Teils während der ganzen Zeit, in der die Substanz appliziert wird, erfolgt.

Der Ausgussadapter ist zwischen dem ersten Folienabschnitt und dem zweiten Folienabschnitt angeordnet und mit diesen fest verbunden. Eine solche Ausgestaltung der Verabreichungsvorrichtung ermöglicht eine effiziente Herstellung und zugleich eine sichere Anwendung.

Dabei umfasst der Ausgussadapter einen Siegelungsabschnitt, der zwischen dem ersten Folienabschnitt und dem zweiten Folienabschnitt angeordnet und mit diesen fest verbunden ist. Ein solcher Siegelungsabschnitt ermöglicht eine effiziente und sichere Verbindung mit den Folienabschnitten. Insbesondere kann er so beschaffen sein, beispielsweise mit Rillen oder einer ähnlichen Struktur, dass die Folienabschnitte sicher verbunden werden können.

Vorzugsweise ist der Anschluss des Ausgussadapters einer Norm entsprechend oder als Luer-Lock-Anschluss ausgestaltet. Ein solcher normierter Anschluss ermöglicht, dass auf einfache effiziente Weise verschiedene passende Teile an der Verabreichungsvorrichtung montiert werden können. Dies ermöglicht, dass diese Teile auf bekannte Art und Weise verbunden werden, was die Handhabung der Verabreichungsvorrichtung vergleichsweise einfach halten kann. Zudem kann mit solchen Anschlüssen auch sicher gestellt werden, dass die Verbindung sicher ist.

Vorzugsweise ist die Kammer im ersten Folienabschnitt ausgeformt. Der zweite Folienabschnitt ist vorzugsweise plan ausgebildet. Der Begriff "plan" kann sich in diesem Zusammenhang auf eine ebene oder flache Form beziehen. Vorzugsweise besteht der erste Folienabschnitt aus einem in einem Tiefziehprozess hergestellten Kunststoff oder aus einem in einem Tiefziehprozess hergestellten Aluminium. Auch der zweite Folienabschnitt kann aus einem solchen Kunststoff beziehungsweise Aluminium hergestellt sein. Das Aluminium kann insbesondere auch ein Aluminiumlaminat sein. Eine solche Ausgestaltung der Folienabschnitte ermöglicht eine effiziente Herstellung der Verabreichungsvorrichtung insbesondere analog der Herstellung bekannter Blisterverpackungen.

Vorzugsweise sind der erste Folienabschnitt als eine erste Folie und der zweite Folienabschnitt als eine zweite Folie ausgebildet. Die zweite Folie kann dabei so hergestellt sein, dass sie für sich alleine nicht formstabil ist. Sie kann eine Abdeckfolie sein, wie sie von herkömmlichen Blisterverpackungen her bekannt ist.

Der erste Folienabschnitt oder der zweite Folienabschnitt oder der erste Folienabschnitt und der zweite Folienabschnitt kann beziehungsweise können mit einer antiseptischen Schicht versehen sein. Eine solche antiseptische Schicht ermöglicht, dass die Substanz in der Verabreichungsvorrichtung steril gehalten werden kann, ohne dass aufwendige weitere Massnahmen getroffen werden müssen. Dies kann insbesondere bei medizinischen Substanzen bevorzugt sein.

Zum Verbinden können der erste und der zweite Folienabschnitt miteinander verklebt, mechanisch verhakt, versiegelt oder verschweisst sein. Vorzugsweise umfasst die Verbindung Siegelnähte. Dabei kann das Versiegeln oder das Verschweissen beispielsweise durch einen thermischen Prozess oder mittels Ultraschall erfolgen. Insbesondere sind die Siegelnähte der Verbindung vorzugsweise durch thermische Siegelung hergestellt.

Der festere Bereich der Verbindung weist bevorzugt Siegelnähte mit höherer Festigkeit und der weniger feste Bereiche der Verbindung Siegelnähte mit tieferer Festigkeit auf. Auf diese Art können die festeren und weniger festen Bereiche der Verbindung effizient in einer angestrebten Qualität hergestellt werden.

Die Verabreichungsvorrichtung kann auch mehrere Kammern aufweisen. Beispielsweise können in den mehreren Kammern Substanzen angeordnet sein, die vor der Verabreichung zusammengemischt werden.

Dazu ist die Verabreichungsvorrichtung vorzugsweise wie folgt ausgestaltet: Der Grundkörper weist eine weitere Kammer zur Aufnahme einer weiteren Substanzkomponente auf. Die Verbindung dichtet die weitere Kammer ab. Der weniger feste Bereich der Verbindung ist zwischen der weiteren Kammer und der Kammer angeordneten. Der weniger feste Bereich der Verbindung ist dazu ausgebildet, sich beim Zusammendrücken der weiteren Kammer zum Austragen der Substanzkomponente aus der weiteren Kammer zu lösen. Der festere Bereich der Verbindung ist dazu ausgebildet, beim Zusammendrücken der weiteren Kammer zum Austragen der Substanzkomponente verbunden zu bleiben. Die Substanzkomponente ist mittels Zusammendrücken der weiteren Kammer quasi ausschliesslich von der weiteren Kammer in die Kammer übertragbar ist.

Unter dem Begriff "Substanzkomponente" kann in diesem Zusammenhang ein Stoff verstanden werden, der zusammen mit einem in der Kammer angeordneten Stoff die Substanz bildet, die am Ende ausgetragen werden soll. Beispielsweise kann die Substanzkomponente Lösungsmittel sein, das von der weiteren Kammer in die Kammer übertragen und dort ein Lyophilisat zur Substanz rekonstituiert. Eine solche Anordnung kann ermöglichen, dass die zu applizierende Substanz erst kurz vor Ausbringung erstellt beziehungsweise gemischt wird und davor hermetisch abgetrennt ist. Dies kann unter anderem aus Haltbarkeitsgründen vorteilhaft sein.

Alternativ dazu kann die Substanzkomponente auch eine weitere Substanz, die zusammen mit der Substanz oder getrennt von ihr ausgegeben wird. So kann eine Anwendung mit mehreren Substanzen effizient ermöglicht werden.

Ein anderer Aspekt der Erfindung betrifft ein Verfahren zur Herstellung einer Verabreichungsvorrichtung insbesondere wie sie oben beschrieben ist. Das Verfahren umfasst die Schritte: (i) Anfertigen eines in einem Tiefziehprozess hergestellten ersten Folienabschnitts; (ii) Ausformen einer Kammer im ersten Folienabschnitt; (iii) Anfertigen eines vorzugsweise in einem Tiefziehprozess hergestellten zweiten Folienabschnitts; (iv) Sterilisieren des ersten Folienabschnitts und des zweiten Folienabschnitts; (v) Positionieren eines Ausgussadapters mit einem Anschluss; (vi) Befüllen der Kammer mit einer sterilen zu verabreichenden fliessfähigen Substanz unter sterilen Bedingungen; (vii) Erstellen einer Verbindung des ersten Folienabschnitts mit dem zweiten Folienabschnitt unter sterilen Bedingungen, sodass der Ausgussadapter dicht mit dem Grundkörper verbunden ist, und die Verbindung mehreren Bereiche aufweist, die einen festeren Bereich und einen zwischen der Kammer und der Auslassöffnung angeordneten weniger festen Bereich umfasst.

Die vorstehenden Schritte des erfindungsgemässen Verfahrens können auch in einer anderen Reihenfolge als wie angegeben (i) - (vii) ausgeführt werden. Auch können mehrere Schritte in einem ausgeführt werde. Beispielsweise kann das Ausformen der Kammer im ersten Folienabschnitt zusammen mit seiner Anfertigung in einer einzigen Schrittkombination erfolgen. Oder die beiden Folienabschnitte können in einem angefertigt werden, was insbesondere bei ersten und zweiten Folienabschnitten in einem Stück zweckmässig sein kann. Das Anfertigen der Folienabschnitte in den Schritten (i) und (iii) kann einem Herstellen des ersten Folienabschnitts beziehungsweise des zweiten Folienabschnitts in einem Tiefziehprozess entsprechen. Die Kammer kann mittels Kaltformen, was beispielsweise bei einem ersten Folienabschnitt aus Aluminium oder Aluminiumlaminat vorteilhaft sein kann, oder Thermoformen, was beispielsweise bei einem ersten Folienabschnitt aus einem Kunststoff vorteilhaft sein kann, ausgeformt werden. Der Tiefziehprozess kann also ein Kaltformen und/oder ein Thermoformen umfassen.

Da die Schritte des Befüllens der Kammer mit der sterilen zu verabreichenden fliessfähigen Substanz und des Schliessens der Kammer durch festes Verbinden des ersten Folienabschnitts mit dem zweiten Folienabschnitt unter sterilen Bedingungen erfolgt, ermöglicht das erfindungsgemässe Verfahren, dass keine zusätzliche Sterilisierung mehr notwendig ist. Insbesondere kann vermieden werden, dass nachgeschaltete Sterilisierungsmassnahmen wie beispielsweise Gammabestrahlung erfolgen müssen. Dadurch kann verhindert werden, dass die Substanz durch diese Massnahmen beeinträchtigt wird. Beispielsweise ermöglicht dies, dass gewisse Substanzen mit biologischen Wirkstoffen überhaupt in einer Verabreichungsvorrichtung der erfindungsgemässen Art bereitgestellt werden kann. Dies ist typischerweise nicht möglich, da die erwähnte zusätzliche Sterilisierung die biologischen Wirkstoffe schädigt. Zudem ermöglicht das erfindungsgemässe Verfahren eine effiziente Herstellung mit verhältnismässig wenigen Prozessschritten beispielsweise in einem sogenannten Inline-Prozess.

Weiter kann mit dem erfindungsgemässen Verfahren auf effiziente Weise die oben beschriebene oder eine ähnliche Verabreichungsvorrichtung beispielsweise in analoger Weise wie herkömmliche Blisterverpackungen hergestellt werden. Dadurch können die Effekte und Vorteile, wie sie oben im Zusammenhang mit der Verabreichungsvorrichtung und derer bevorzugter Ausführungsformen beschrieben sind, effizient implementiert werden.

Dabei wird vorzugsweise ein Ausgussadapter angrenzend an die befüllte Kammer angeordnet, wobei beim Schliessen der Kammer der Ausgussadapter fest mit dem ersten Folienabschnitt und dem zweiten Folienabschnitt verbunden wird. Zeitlich kann sich der Begriff "beim Schliessen der Kammer" auf ein Verbinden sowohl während dem Schliessen der Kammer als auch vor oder nach dem Schliessen der Kammer beziehen. Dies ermöglicht eine besonders einfache und effiziente Implementierung des Ausgussadapters in der Verabreichungsvorrichtung.

Auch ist dabei der weniger feste Bereich der Verbindung dazu ausgebildet, sich beim Zusammendrücken der Kammer zum Austragen der Substanz durch die Auslassöffnung zu lösen, und der festere Bereich der Verbindung dazu ausgebildet, beim Zusammendrücken der Kammer zum Austragen der Substanz durch die Auslassöffnung verbunden zu bleiben, sodass die Substanz mittels Zusammendrücken der Kammer durch die Auslassöffnung quasi ausschliesslich durch die Auslassöffnung und durch den Anschluss des Ausgussadapters austragbar ist.

Nachfolgend werden einige bevorzugte Ausführungsformen des erfindungsgemässen Verfahrens beschrieben, die zum Erreichen der Effekte und Vorteile der oben beschriebenen entsprechenden Ausführungsformen der erfindungsgemässen Verabreichungsvorrichtung und/oder zum Erreichen weiterer Effekte und Vorteile vorgesehen sein können.

Der Ausgussadapter kann aus einem festen Material hergestellt sein, das bei einem Zusammendrücken der Kammer zum Austragen der Substanz durch die Auslassöffnung im Wesentlichen seine Form beibehält.

Der Ausgussadapter wird beim Schliessen der Kammer durch festes Verbinden des ersten Folienabschnitts mit dem zweiten Folienabschnitt zwischen dem ersten Folienabschnitt und dem zweiten Folienabschnitt angeordnet und mit diesen fest verbunden. Dazu umfasst der Ausgussadapter einen Siegelungsabschnitt, der zwischen dem ersten Folienabschnitt und dem zweiten Folienabschnitt angeordnet und mit diesen fest verbunden ist.

Zeitlich wird der Ausgussadapter bevorzugt vor dem Schliessen der Kammer mit dem ersten und/oder zweiten Folienabschnitt verbunden.

Vorzugsweise wird der Ausgussadapter beim Positionieren und vor dem Befüllen der Kammer am ersten Folienabschnitt befestigt.

Der erste Folienabschnitt kann aus einem in einem Tiefziehprozess hergestellten Kunststoff oder aus einem in einem Tiefziehprozess hergestellten Aluminium bestehen. Der zweite Folienabschnitt kann plan ausgebildet sein.

Der erste Folienabschnitt kann als eine erste Folie und der zweite Folienabschnitt als eine zweite Folie ausgebildet werden, wobei die erste Folie mit der zweiten Folie fest verbunden wird. Der zweite Folienabschnitt kann aus einer Aluminiumfolie hergestellt sein.

Der erste Folienabschnitt oder der zweite Folienabschnitt oder der erste Folienabschnitt und der zweite Folienabschnitt kann mit einer antiseptischen Schicht versehen werden.

Vorzugsweise wird die Verbindung des ersten Folienabschnitts mit dem zweiten Folienabschnitt unter sterilen Bedingungen durch thermische Siegelung erstellt. Ein solcher Verbindungsschritt kann schnell und kosteneffizient durchgeführt werden, wobei eine sichere Verbindung erstellt werden kann. Die Verbindung wird vorzugsweise durch Siegelnähte bevorzugt mittels thermischer Siegelung erstellt. Dabei werden festere Bereich der Verbindung durch Siegelnähte mit höherer Festigkeit und der weniger feste Bereiche der Verbindung durch Siegelnähte mit tieferer Festigkeit erstellt. Die unterschiedlichen Siegelnähte können durch unterschiedliche Prozessparameter wie beispielsweise den Druck, die Temperatur oder die Dauer erzeugt werden.

Vorzugsweise ist der Anschluss des Ausgussadapters einer Norm entsprechend oder als Luer-Lock-Anschluss ausgestaltet ist.

### Kurze Beschreibung der Zeichnungen

Weitere vorteilhafte Ausgestaltungen der Erfindung ergeben sich aus der nachfolgenden Beschreibung eines Ausführungsbeispiels der Erfindung mit Hilfe der schematischen Zeichnungen. Insbesondere wird im Folgenden die erfindungsgemässe Verabreichungsvorrichtung unter Bezugnahme auf die beigefügten Zeichnungen anhand des Ausführungsbeispiels detaillierter beschrieben. Es zeigen:
- Fig. 1: eine schematische Aufsicht eines Dispensers als Ausführungsbeispiel einer erfindungsgemässen Verabreichungsvorrichtung;
- Fig. 2: eine Seitenansicht des Dispenser von Fig. 1;
- Fig. 3: eine erste perspektivische Ansicht auf die eine der Oberflächen des Dispensers von Fig. 1; und
- Fig. 4: eine zweite perspektivische Ansicht auf die gegenüberliegende andere der Oberflächen des Dispensers von Fig. 1.

### Weq(e) zur Ausführung der Erfindung

Bestimmte Ausdrücke werden in der folgenden Beschreibung aus praktischen Gründen verwendet und sind nicht einschränkend zu verstehen. Die Wörter "rechts", "links", "unten" und "oben" bezeichnen Richtungen in der Zeichnung, auf die Bezug genommen wird. Die Ausdrücke "nach innen", "nach aussen" "unterhalb", "oberhalb", "links", "rechts" oder ähnliche werden zur Beschreibung der Anordnung bezeichneter Teile zueinander, der Bewegung bezeichneter Teile zueinander und der Richtungen hin zum oder weg vom geometrischen Mittelpunkt der Erfindung sowie benannter Teile derselben wie in den Fig. dargestellt verwendet. Diese räumlichen Relativangaben umfassen auch andere Positionen und Ausrichtungen als die in den Fig. dargestellten. Zum Beispiel wenn ein in den Fig. dargestelltes Teil umgedreht wird, sind Elemente oder Merkmale, die als "unterhalb" beschrieben sind, dann "oberhalb". Die Terminologie umfasst die oben ausdrücklich erwähnten Wörter, Ableitungen von denselben und Wörter ähnlicher Bedeutung.

Um Wiederholungen in den Fig. und der zugehörigen Beschreibung der verschiedenen Aspekte und Ausführungsbeispielen zu vermeiden, sollen bestimmte Merkmale als gemeinsam für verschieden Aspekte und Ausführungsbeispiele verstanden werden. Das Weglassen eines Aspekts in der Beschreibung oder einer Fig. lässt nicht darauf schliessen, dass dieser Aspekt in dem zugehörigen Ausführungsbeispiel fehlt. Vielmehr kann ein solches Weglassen der Klarheit und dem Verhindern von Wiederholungen dienen. In diesem Zusammenhang gilt für die gesamte weitere Beschreibung folgende Festlegung: Sind in einer Figur zum Zweck zeichnerischer Eindeutigkeit Bezugszeichen enthalten, aber im unmittelbar zugehörigen Beschreibungstext nicht erwähnt, so wird auf deren Erläuterung in vorangehenden Figurenbeschreibungen Bezug genommen. Sind ausserdem im unmittelbar zu einer Figur gehörigen Beschreibungstext Bezugszeichen erwähnt, die in der zugehörigen Figur nicht enthalten sind, so wird auf die vorangehenden und nachstehenden Figuren verwiesen. Ähnliche Bezugszeichen in zwei oder mehreren Fig. stehen für ähnliche oder gleiche Elemente.

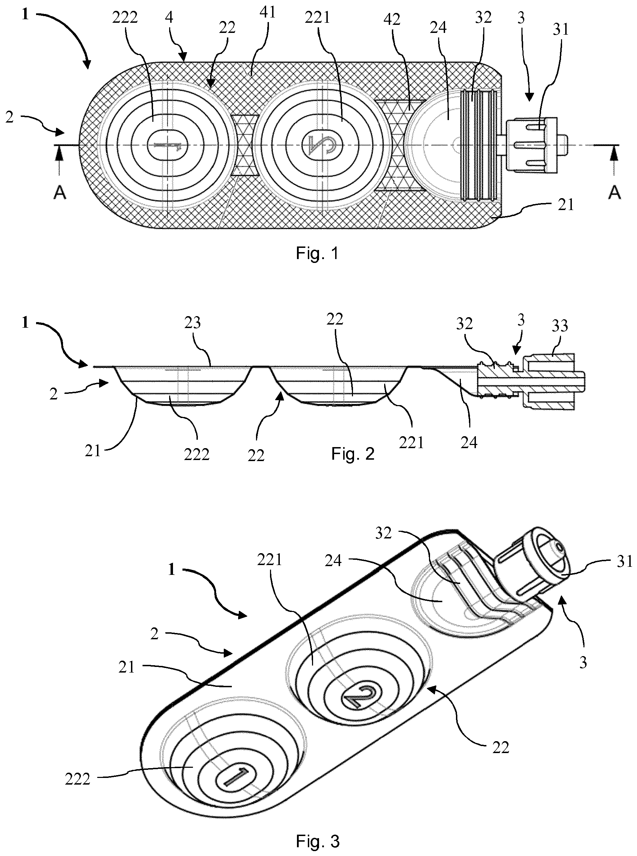

Fig. 1 zeigt einen Dispenser 1 als ein Ausführungsbeispiel einer erfindungsgemässen Verabreichungsvorrichtung. Der Dispenser 1 ist dabei als Portionenpackung ausgebildet. Er umfasst einen formstabilen, manuell deformierbaren Grundkörper 2, der eine erste Folie 21 als ersten Folienabschnitt aufweist. In der ersten Folie 21 sind zwei Kammern 22 und eine Auslassöffnung 24 ausgebildet. In einer rechten ersten Kammer 221 ist eine Portion eines lyophilisierten pharmazeutischen Wirkstoffs angeordnet und in der linken zweiten Kammer 222 ein flüssiges Lösungsmittel. Die Auslassöffnung 24 umfasst eine am rechten Rand des Grundkörpers 2 ausgebildete Auslasskammer. Der Grundkörper 2 hat in der Aufsicht eine etwa rechteckige Grundform. Die erste Folie 21 besteht aus einem in einem Tiefziehprozess hergestellten Aluminiumlaminat.

Der Dispenser 1 umfasst weiter einen Ausgussadapter 3 mit einem Luer-Lock-Anschluss 31 und einem strukturierten Siegelungsabschnitt 32. Er ist an seinem Siegelungsabschnitt 32 mit dem Grundkörper fest 2 verbunden, sodass der Luer-Lock-Anschluss von einer der kürzeren Querseiten des Grundkörpers 2 absteht.

In Fig. 2 ist der Dispenser 1 von der Seite her gezeigt. Der Grundkörper 2 ist neben der ersten Folie 21 aus einer zweiten Folie 23 als zweiten Folienabschnitt hergestellt. Die zweite Folie 23 ist aus Aluminiumlaminat hergestellt und eben ausgestaltet. Die Kammern 22 sind als sich nach unten erstreckende Ausbuchtungen in der ersten Folie 21 ausgebildet. Dabei befindet sich die zweite Folie 23 auf der ersten Folie 21 und ist mit diesem über eine aus Siegelnähten gebildeten, in der Fig. 1 dargestellten Verbindung 4 fest und dicht verbunden. Die Kammern 22 sind so hermetisch verschlossen.

Die Verbindung 4 umfasst einen festeren Bereich 41 und einen weniger festen Bereich 42. Der festere Bereich 41 ist insbesondere zwischen den Kammern 22 und der Peripherie der Grundkörpers 2 angeordnet. Der weniger feste Bereich 42 der Verbindung 4 ist zwischen der ersten Kammer 221 und der Auslasskammer der Auslassöffnung 24 sowie zwischen der ersten Kammer 221 und der zweiten Kammer 222 angeordnet.

Wie in Fig. 2 ersichtlich ist, weist der Ausgussadapter 3 einen horizontalen Kanal auf. Der Kanal bildet eine Auslassöffnung des Dispensers 1, der an ein am Luer-Lock-Anschluss montiertes passendes Teil (in den Fig. nicht dargestellt) übergeht.

Fig. 3 zeigt eine perspektivische Ansicht des Dispensers 1 von der ersten Folie 21 her. Der Siegelungsabschnitt 32 des Ausgussadapters 3 hat eine in der Frontansicht quasi dreieckige Form, wobei der Luer-Lock-Anschluss 31 mittig davon absteht. Der Siegelungsabschnitt 32 ist zwischen der ersten Folie 21 und der zweiten Folie 23 angeordnet und fest und dicht mit diesen verbunden.

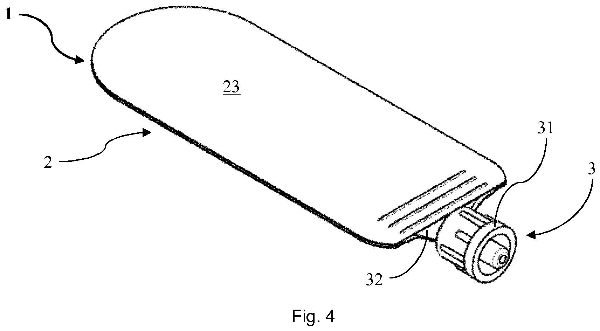

Wie in Fig. 4 ersichtlich ist, bildet die zweite Folie 23 eine komplett ebene beziehungsweise plane Oberfläche des Grundkörpers 2 des Dispensers 1. Der Ausguss 3 ist aus einem festen Material hergestellt, das bei einem Zusammendrücken der Kammern 22 zur Verabreichung der Substanz beziehungsweise des Medikaments durch den Ausgussadapter 3 im Wesentlichen seine Form beibehält.

In Anwendung des Dispensers 1 wird ein passendes Teil wie beispielsweise eine Injektionsnadel am Luer-Lock-Anschluss 31 montiert. Dann wird in einem ersten Schritt die zweite Kammer 222, die zur Illustration mit einer Zahl eins ausgestattet ist, zusammengedrückt. Durch den in der zweiten Kammer 222 dadurch erzeugten Überdruck wird der weniger feste Bereich 42 zwischen erster Kammer 221 und zweiter Kammer 222 gelöst, sodass ein Durchgang zwischen diesen beiden Kammern entsteht. Das Lösungsmittel wird in die erste Kammer 221 gefördert, wo es das lyophilisierte Medikament zur zu verabreichenden Substanz rekonstituiert. Die Injektionsnadel wird dann an einem Patienten appliziert und die erste Kammer 221 wird zusammengedrückt. Dadurch wird ein Überdruck in der ersten Kammer 221 erzeugt mittels dem der weniger feste Bereich 42 zwischen erster Kammer 221 und Auslassöffnung 24 gelöst wird, sodass ein Durchgang zwischen diesen beiden Kammern entsteht. Die Substanz wird dann durch die Auslassöffnung 24, den Ausgussadapter 3 und die Injektionsnadel ausgetragen und injiziert. Um dabei einen Rückfluss der Substanz von der ersten Kammer 221 in die zweite Kammer 222 zu verhindern wird entweder die zweite Kammer 222 zusammengehalten, während die erste Kammer zusammengedrückt wird, oder der Grundkörper 2 wird zwischen erster Kammer 221 und zweiter Kammer 222 umgefaltet.

Der Dispenser 1 kann wie folgt analog einer herkömmlichen Blisterverpackung hergestellt sein. Die erste Folie 21 wird in einem Tiefziehprozess hergestellt, wobei die Kammern 22 dabei im ersten Folienabschnitt beispielsweise mittels Thermoformen ausgeformt werden. Die zweite Folie 23 wird hergestellt beziehungsweise hergerichtet. Die erste Folien 21 und die zweite Folie 23 werden sterilisiert und die Kammern 22 werden mit den zugehörigen Substanzkomponenten unter sterilen Bedingungen befüllt. Der vorgefertigte Ausgussadapter 3 wird angrenzend an die befüllte Kammer 22 angeordnet. Die Kammer 22 wird dann durch festes Verbinden der ersten Folie 21 mit der zweiten Folie 23 unter sterilen Bedingungen geschlossen, wobei gleichzeitig der Siegelungsabschnitt 32 des Ausgusses 3 mit den Folien 21, 23 fest und dicht verbunden wird.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Um die Erfindung nicht zu verklären, können in gewissen Fällen wohlbekannte Strukturen und Techniken nicht im Detail gezeigt und beschrieben sein. Es versteht sich, dass Fachleute Änderungen und Abwandlungen machen können, ohne den Umfang der folgenden Ansprüche zu verlassen.

Im Weiteren schliesst der Ausdruck "umfassen" und Ableitungen davon andere Elemente oder Schritte nicht aus. Ebenfalls schliesst der unbestimmte Artikel "ein" bzw. "eine" und Ableitungen davon eine Vielzahl nicht aus. Die Funktionen mehrerer in den Ansprüchen aufgeführter Merkmale können durch eine Einheit beziehungsweise einen Schritt erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Die Begriffe "etwa" und "ungefähr" im Zusammenhang mit einem gegebenen Zahlenwert oder -bereich kann sich auf einen Wert beziehungsweise Bereich beziehen, der innerhalb 20%, innerhalb 10%, innerhalb 5% oder innerhalb 2% des gegebenen Werts beziehungsweise Bereichs liegt. Alle Bezugszeichen in den Ansprüchen sind nicht als den Umfang der Ansprüche einschränkend zu verstehen.

## Patentansprüche

1. Verabreichungsvorrichtung (1) mit einem Grundkörper (2), der
eine Auslassöffnung (24) und eine Kammer (22) zur Aufnahme einer zu verabreichenden fliessfähigen Substanz aufweist,
aus einem vorzugsweise als eine erste Folie (21) ausgebildeten ersten Folienabschnitt (21) und einem mit dem ersten Folienabschnitt (21) verbundenen vorzugsweise als eine zweite Folie (23) ausgebildeten zweiten Folienabschnitt (23), der vorzugsweise plan ausgebildet ist, hergestellt ist, und
dazu ausgestaltet ist, dass die Substanz mittels Zusammendrücken der Kammer (22) durch die Auslassöffnung (24) austragbar ist,
**gekennzeichnet durch**
einen dicht mit der Auslassöffnung (24) verbundenen Ausgussadapter (3) mit einem Anschluss (31), wobei
die Verbindung (4) des ersten Folienabschnitts (21) mit dem zweiten Folienabschnitt (23) die Kammer (22) abdichtet und mehrer Bereiche aufweist,
die mehreren Bereiche der Verbindung (4) einen festeren Bereich (41) und einen zwischen der Kammer (22) und der Auslasskammer der Auslassöffnung (24) angeordneten weniger festen Bereich (42) umfassen,
der weniger feste Bereich (42) der Verbindung (4) dazu ausgebildet ist, sich beim Zusammendrücken der Kammer (22) zum Austragen der Substanz durch die Auslasskammer der Auslassöffnung (24) zu lösen, und
der festere Bereich (41) der Verbindung (4) dazu ausgebildet ist, beim Zusammendrücken der Kammer (22) zum Austragen der Substanz durch die Auslasskammer der Auslassöffnung (24) verbunden zu bleiben,
sodass die Substanz mittels Zusammendrücken der Kammer (22) quasi ausschliesslich durch die Auslasskammer der Auslassöffnung (24) und durch den Anschluss (31) des Ausgussadapters (3) austragbar ist, wobei
ein passend auf die gewünschte Verabreichungsart der Substanz, wie Spritzen, Tropfen, Aufstreichen oder Injizieren, passendes Teil am Anschluss des Ausgussadapters montierbar ist, wobei das Teil einen Ausguss, einen Tröpfchenspender oder eine Injektionsnadel einer gewünschten Länge umfasst, und wobei Ausgussadapter (3) einen Siegelungsabschnitt (32) aufweist,
der zwischen der ersten Folie (21) und der zweiten Folie (23) angeordnet und mit diesen fest verbunden ist,
**dadurch gekennzeichnet, dass** der Siegelungsabschnitt (32) in der Frontansicht eine quasi dreieckige Form hat.

2. Verabreichungsvorrichtung (1) nach Anspruch 1, bei welcher
der Ausgussadapter (3) aus einem festen Material hergestellt ist, das beim Zusammendrücken der Kammer (22) zum Austragen der Substanz im Wesentlichen seine Form beibehält, und/oder
der Anschluss (31) des Ausgussadapters (3) einer Norm entsprechend oder als Luer-Lock-Anschluss (31) ausgestaltet ist.

3. Verabreichungsvorrichtung (1) nach Anspruch 1 oder 2, bei der die Kammer (22) im ersten Folienabschnitt (21) ausgeformt ist.

4. Verabreichungsvorrichtung (1) nach einem der vorangehenden Ansprüche, bei welcher
der erste Folienabschnitt (21) aus einem in einem Tiefziehprozess hergestellten Kunststoff oder aus einem in einem Tiefziehprozess hergestellten Aluminium besteht und/oder
der zweite Folienabschnitt (23) aus einer Aluminiumfolie hergestellt ist.

5. Verabreichungsvorrichtung (1) nach einem der vorangehenden Ansprüche, bei welcher der erste Folienabschnitt (21) oder der zweite Folienabschnitt (23) oder der erste Folienabschnitt (21) und der zweite Folienabschnitt (23) mit einer antiseptischen Schicht versehen ist.

6. Verabreichungsvorrichtung (1) nach einem der vorangehenden Ansprüche, bei der die Verbindung (4) Siegelnähte umfasst, wobei vorzugsweise die Siegelnähte der Verbindung (4) durch thermische Siegelung hergestellt sind und/oder der festere Bereich (41) der Verbindung (4) Siegelnähte mit höherer Festigkeit und der weniger feste Bereich (42) der Verbindung (4) Siegelnähte mit tieferer Festigkeit aufweisen.

7. Verabreichungsvorrichtung (1) nach einem der vorangehenden Ansprüche, bei welcher
der Grundkörper eine weitere Kammer zur Aufnahme einer weiteren Substanzkomponente aufweist,
die Verbindung (4) die weitere Kammer abdichtet,
der weniger feste Bereich (42) der Verbindung (4) zwischen der weiteren Kammer und der Kammer (22) angeordneten ist,
der weniger feste Bereich (42) der Verbindung (4) dazu ausgebildet ist, sich beim Zusammendrücken der weiteren Kammer zum Austragen der Substanzkomponente aus der weiteren Kammer zu lösen, und
der festere Bereich (41) der Verbindung (4) dazu ausgebildet ist, beim Zusammendrücken der weiteren Kammer zum Austragen der Substanzkomponente verbunden zu bleiben,
sodass die Substanzkomponente mittels Zusammendrücken der weiteren Kammer quasi ausschliesslich von der weiteren Kammer in die Kammer (22) übertragbar ist.

8. Verfahren zur Herstellung einer Verabreichungsvorrichtung (1) insbesondere nach einem der vorangehenden Ansprüche, umfassend:
Anfertigen eines in einem Tiefziehprozess hergestellten ersten Folienabschnitts (21);
Ausformen einer Kammer (22) im ersten Folienabschnitt (21);
Anfertigen eines vorzugsweise in einem Tiefziehprozess hergestellten zweiten Folienabschnitts (23), der vorzugsweise aus einer Aluminiumfolie hergestellt ist und der vorzugsweise plan ausgebildet ist;
Sterilisieren des ersten Folienabschnitts (21) und des zweiten Folienabschnitts (23);
Positionieren eines Ausgussadapters (3) mit einem Anschluss;
Befüllen der Kammer (22) mit einer sterilen zu verabreichenden fliessfähigen Substanz unter sterilen Bedingungen;
Erstellen einer Verbindung (4) des ersten Folienabschnitts (21) mit dem zweiten Folienabschnitt (23) unter sterilen Bedingungen, sodass
der Ausgussadapter (3) dicht mit dem Grundkörper verbunden ist, und
die Verbindung (4) mehrere Bereiche aufweist, die einen festeren Bereich (41) und einen zwischen der Kammer (22) und der Auslasskammer der Auslassöffnung (24) angeordneten weniger festen Bereich (42) umfasst, wobei
der weniger feste Bereich (42) der Verbindung (4) dazu ausgebildet ist, sich beim Zusammendrücken der Kammer (22) zum Austragen der Substanz durch die Auslasskammer der Auslassöffnung (24) zu lösen, und
der festere Bereich (41) der Verbindung (4) dazu ausgebildet ist, beim Zusammendrücken der Kammer (22) zum Austragen der Substanz durch die Auslasskammer der Auslassöffnung (24) verbunden zu bleiben,
sodass die Substanz mittels Zusammendrücken der Kammer (22) durch die Auslasskammer der Auslassöffnung (24) quasi ausschliesslich durch die Auslassöffnung (24) und durch den Anschluss (31) des Ausgussadapters (3) austragbar ist, wobei
ein passend auf die gewünschte Verabreichungsart der Substanz, wie Spritzen, Tropfen, Aufstreichen oder Injizieren, passendes Teil am Anschluss des Ausgussadapters montierbar ist, wobei das Teil einen Ausguss, einen Tröpfchenspender oder eine Injektionsnadel einer gewünschten Länge umfasst, und wobei Ausgussadapter (3) einen Siegelungsabschnitt (32) aufweist, welcher in der Frontansicht eine quasi dreieckige Form hat, und wobei
der Siegelungsabschnitt (32) zwischen der ersten Folie (21) und der zweiten Folie (23) angeordnet und mit diesen fest verbunden ist.

9. Verfahren nach Anspruch 8, bei dem der Ausgussadapter (3)
aus einem festen Material hergestellt ist, das bei einem Zusammendrücken der Kammer (22) zum Austragen der Substanz aus der Kammer (22) im Wesentlichen seine Form beibehält, und/oder
beim Schliessen der Kammer (22) durch festes Verbinden des ersten Folienabschnitts (21) mit dem zweiten Folienabschnitt (23) zwischen dem ersten Folienabschnitt (21) und dem zweiten Folienabschnitt (23) angeordnet und mit diesen dicht verbunden wird, wobei der Ausgussadapter (3) vorzugsweise einen Siegelungsabschnitt aufweist, der zwischen dem ersten Folienabschnitt (21) und dem zweiten Folienabschnitt (23) angeordnet und mit diesen fest verbunden ist, und/oder
beim Positionieren und vor dem Befüllen der Kammer am ersten Folienabschnitt befestigt wird.

10. Verfahren nach einem der Ansprüche 8 bis 9, bei dem der erste Folienabschnitt (21) aus einem in einem Tiefziehprozess hergestellten Kunststoff oder aus einem in einem Tiefziehprozess hergestellten Aluminium besteht.

11. Verfahren nach einem der Ansprüche 8 bis 10, bei dem
der erste Folienabschnitt (21) als eine erste Folie (21) und der zweite Folienabschnitt (23) als eine zweite Folie (23) ausgebildet werden, wobei die erste Folie (21) mit der zweiten Folie (23) fest verbunden wird, und/oder
der erste Folienabschnitt (21) oder der zweite Folienabschnitt (23) oder der erste Folienabschnitt (21) und der zweite Folienabschnitt (23) mit einer antiseptischen Schicht versehen wird.

12. Verfahren nach einem der Ansprüche 8 bis 11, bei dem die Verbindung (4) des ersten Folienabschnitts (21) mit dem zweiten Folienabschnitt (23) unter sterilen Bedingungen durch thermische Siegelung erstellt wird.

13. Verfahren nach einem der Ansprüche 8 bis 12, bei dem der Anschluss (31) des Ausgussadapters (3) einer Norm entsprechend oder als Luer-Lock-Anschluss (31) ausgestaltet ist.

14. Verfahren nach einem der Ansprüche 8 bis 13, bei dem die Verbindung (4) durch Siegelnähte erstellt wird, wobei vorzugsweise die Siegelnähte der Verbindung (4) durch thermische Siegelung hergestellt werden und/oder der festere Bereich (41) der Verbindung (4) durch Siegelnähte mit höherer Festigkeit und der weniger feste Bereich (42) der Verbindung (4) durch Siegelnähte mit tieferer Festigkeit erstellt werden.

## Claims

1. Administering device (1) comprising a main body (2), which
has an outlet opening (24) and a chamber (22) for receiving a flowable substance to be administered,
is made of a first film portion (21), which is preferably in the form of a first film (21), and a second film portion (23), which is connected to the first film portion (21) and is preferably in the form of a second film (23) and is preferably planar, and
is designed such that the substance can be discharged through the outlet opening (24) by compressing the chamber (22),
**characterized by**
a pouring adapter (3), which is tightly connected to the outlet opening (24) and has a connector (31), wherein
the connection (4) between the first film portion (21) and the second film portion (23) seals off the chamber (22) and has a plurality of regions,
the plurality of regions of the connection (4) comprise a more rigid region (41) and a less rigid region (42) arranged between the chamber (22) and the outlet chamber of the outlet opening (24),
the less rigid region (42) of the connection (4) is designed to detach when the chamber (22) is compressed for discharging the substance through the outlet chamber of the outlet opening (24), and
the more rigid region (41) of the connection (4) is designed to remain connected when the chamber (22) is compressed for discharging the substance through the outlet chamber of the outlet opening (24)
and so, by compressing the chamber (22), the substance can be discharged virtually exclusively through the outlet chamber of the outlet opening (24) and through the connector (31) of the pouring adapter (3), wherein
a part matching the desired administration type of the substance, such as syringing, dropping, applying or injecting, can be mounted on the connector of the pouring adapter, wherein the part comprises a pouring means, a droplet dispenser or an injection needle of a desired length, and wherein the pouring adapter (3) has a sealing portion (32), which is arranged between the first film (21) and the second film (23) and is rigidly connected thereto,
**characterized in that,** when viewed from the front, the sealing portion (32) has a virtually triangular shape.

2. Administering device (1) according to claim 1, wherein
the pouring adapter (3) is made of a rigid material which substantially maintains its shape when the chamber (22) is compressed for discharging the substance, and/or
the connector (31) of the pouring adapter (3) is designed to correspond to a standard or is designed as a luer lock connector (31).

3. Administering device (1) according to claim 1 or 2, wherein the chamber (22) is formed in the first film portion (21).

4. Administering device (1) according to any of the preceding claims, wherein
the first film portion (21) consists of a plastics material produced in a thermoforming process or of an aluminum produced in a deep-drawing process, and/or
the second film portion (23) is made of an aluminum foil.

5. Administering device (1) according to any of the preceding claims, wherein the first film portion (21) or the second film portion (23), or the first film portion (21) and the second film portion (23), is provided with an antiseptic layer.

6. Administering device (1) according to any of the preceding claims, in which the connection (4) comprises sealing seams, wherein the sealing seams of the connection (4) are preferably produced by thermal sealing and/or the more rigid region (41) of the connection (4) has sealing seams having higher rigidity and the less rigid region (42) of the connection (4) has sealing seams having lower rigidity.

7. Administering device (1) according to any of the preceding claims, wherein
the main body has a further chamber for receiving a further substance component,
the connection (4) seals off the further chamber,
the less rigid region (42) of the connection (4) is arranged between the further chamber and the chamber (22),
the less rigid region (42) of the connection (4) is designed to detach when the further chamber is compressed for discharging the substance component from the further chamber, and
the more rigid region (41) of the connection (4) is designed to remain connected when the further chamber is compressed for discharging the substance component
and so, by compressing the further chamber, the substance component can be transferred virtually exclusively from the further chamber into the chamber (22).

8. Method for producing an administering device (1), in particular according to any of the preceding claims, comprising:
making a first film portion (21) produced in a thermoforming process;
shaping a chamber (22) in the first film portion (21);
making a second film portion (23) preferably produced in a deep-drawing process, which second film portion is preferably made of an aluminum foil and is preferably planar;
sterilizing the first film portion (21) and the second film portion (23);
positioning a pouring adapter (3) having a connector;
filling the chamber (22) under sterile conditions with a flowable substance to be administered sterile;
creating a connection (4) between the first film portion (21) and the second film portion (23) under sterile conditions so that
the pouring adapter (3) is tightly connected to the main body, and
the connection (4) has a plurality of regions comprising a more rigid region (41) and a less rigid region (42) arranged between the chamber (22) and the outlet chamber of the outlet opening (24), wherein
the less rigid region (42) of the connection (4) is designed to detach when the chamber (22) is compressed for discharging the substance through the outlet chamber of the outlet opening (24), and
the more rigid region (41) of the connection (4) is designed to remain connected when the chamber (22) is compressed for discharging the substance through the outlet chamber of the outlet opening (24)
and so, by compressing the chamber (22), the substance can be discharged through the outlet chamber of the outlet opening (24) virtually exclusively through the outlet opening (24) and through the connector (31) of the pouring adapter (3), wherein
a part matching the desired administration type of the substance, such as syringing, dropping, applying or injecting, can be mounted on the connector of the pouring adapter, wherein the part comprises a pouring means, a droplet dispenser or an injection needle of a desired length, and wherein the pouring adapter (3) has a sealing portion (32) which, when viewed from the front, has a virtually triangular shape, and wherein
the sealing portion (32) is arranged between the first film (21) and the second film (23) and is rigidly connected thereto.

9. Method according to claim 8, in which the pouring adapter (3)
is made of a solid material which substantially maintains its shape when the chamber (22) is compressed for discharging the substance from the chamber (22), and/or
is arranged between the first film portion (21) and the second film portion (23) and is tightly connected thereto when the chamber (22) is closed by rigid connecting of the first film portion (21) to the second film portion (23), wherein the pouring adapter (3) preferably has a sealing portion which is arranged between the first film portion (21) and the second film portion (23) and is rigidly connected thereto, and/or
is fastened to the first film portion during positioning and prior to filling of the chamber.

10. Method according to any of claims 8 to 9, wherein the first film portion (21) consists of a plastics material produced in a thermoforming process or of an aluminum produced in a deep-drawing process.

11. Method according to any of claims 8 to 10, in which
the first film portion (21) is in the form of a first film (21) and the second film portion (23) is in the form of a second film (23), wherein the first film (21) is rigidly connected to the second film (23), and/or
the first film portion (21) or the second film portion (23), or the first film portion (21) and the second film portion (23), is provided with an antiseptic layer.

12. Method according to any of claims 8 to 11, wherein the connection (4) between the first film portion (21) and the second film portion (23) is created under sterile conditions by thermal sealing.

13. Method according to any of claims 8 to 12, wherein the connector (31) of the pouring adapter (3) is designed to correspond to a standard or is designed as a luer lock connection (31).

14. Method according to any of claims 8 to 13, in which the connection (4) is produced by sealing seams, wherein the sealing seams of the connection (4) are preferably produced by thermal sealing and/or the more rigid region (41) of the connection (4) is produced by sealing seams having higher rigidity and the less rigid region (42) of the connection (4) is produced by sealing seams having lower rigidity.

## Revendications

1. Dispositif d'administration (1) comportant un corps de base (2) qui
présente une ouverture de sortie (24) et une chambre (22) pour la réception d'une substance fluide à administrer,
est fabriqué à partir d'une première section de film (21) conçue de préférence sous la forme d'un premier film (21) et d'une seconde section de film (23) reliée à la première section de film (21) et conçue de préférence sous la forme d'un second film (23), laquelle seconde section de film est de préférence conçue de manière plane, et
est réalisé de manière à ce que la substance puisse être évacuée, au moyen de la compression de la chambre (22), à travers l'ouverture de sortie (24),
**caractérisé par**
un adaptateur de bec verseur (3) relié de manière étanche à l'ouverture de sortie (24) et comportant un raccord (31), dans lequel
la liaison (4) de la première section de film (21) à la seconde section de film (23) assure l'étanchéité de la chambre (22) et présente plusieurs zones,
les zones de la liaison (4) comprennent une zone plus solide (41) et une zone moins solide (42) agencée entre la chambre (22) et la chambre de sortie de l'ouverture de sortie (24),
la zone moins solide (42) de la liaison (4) est conçue pour se détacher lors de la compression de la chambre (22) pour l'évacuation de la substance à travers la chambre de sortie de l'ouverture de sortie (24), et
la zone plus solide (41) de la liaison (4) est conçue pour rester reliée lors de la compression de la chambre (22) pour l'évacuation de la substance à travers la chambre de sortie de l'ouverture de sortie (24),
de sorte que la substance, au moyen de la compression de la chambre (22), peut être évacuée quasiment exclusivement à travers la chambre de sortie de l'ouverture de sortie (24) et à travers le raccord (31) de l'adaptateur de bec verseur (3), dans lequel
une pièce adaptée, qui est adaptée au mode d'administration souhaité de la substance, comme la projection, goutte-à-goutte, application ou injection, peut être montée sur le raccord de l'adaptateur de bec verseur, dans lequel la pièce comprend un bec verseur, un distributeur de gouttelettes ou une aiguille d'injection d'une longueur souhaitée, et dans lequel l'adaptateur de bec verseur (3) présente une section de scellement (32) qui est agencée entre le premier film (21) et le second film (23) et reliée de manière fixe à ceux-ci,
**caractérisé en ce que** la section de scellement (32) possède une forme quasiment triangulaire en vue de face.

2. Dispositif d'administration (1) selon la revendication 1, dans lequel
l'adaptateur de bec verseur (3) est fabriqué à partir d'un matériau solide qui, lors de la compression de la chambre (22) pour l'évacuation de la substance, retient sensiblement sa forme, et/ou
le raccord (31) de l'adaptateur de bec verseur (3) est conforme à une norme ou est réalisé comme un raccord Luer-Lock (31).

3. Dispositif d'administration (1) selon la revendication 1 ou 2, dans lequel la chambre (22) est formée dans la première section de film (21).

4. Dispositif d'administration (1) selon l'une des revendications précédentes, dans lequel
la première section de film (21) est constituée d'une matière plastique fabriquée dans un processus d'emboutissage profond ou d'un aluminium fabriqué dans un processus d'emboutissage profond et/ou
la seconde section de film (23) est fabriquée à partir d'un film d'aluminium.

5. Dispositif d'administration (1) selon l'une des revendications précédentes, dans lequel la première section de film (21) ou la seconde section de film (23) ou la première section de film (21) et la seconde section de film (23) sont pourvues d'une couche antiseptique.

6. Dispositif d'administration (1) selon l'une des revendications précédentes, dans lequel la liaison (4) comprend des joints scellés, dans lequel de préférence les joints scellés de la liaison (4) sont fabriqués par scellement thermique et/ou la zone plus solide (41) de la liaison (4) présente des joints scellés comportant une plus grande résistance et la zone moins solide (42) de la liaison (4) présente des joints scellés comportant une plus faible résistance.

7. Dispositif d'administration (1) selon l'une des revendications précédentes, dans lequel
le corps de base présente une autre chambre pour la réception d'un autre composant de substance,
la liaison (4) assure l'étanchéité de l'autre chambre,
la zone moins solide (42) de la liaison (4) est agencée entre l'autre chambre et la chambre (22),
la zone moins solide (42) de la liaison (4) est conçue pour se détacher lors de la compression de l'autre chambre pour l'évacuation du composant de substance depuis l'autre chambre, et
la zone plus solide (41) de la liaison (4) est conçue pour rester reliée lors de la compression de l'autre chambre pour l'évacuation du composant de substance,
de sorte que le composant de substance peut être transféré quasiment exclusivement depuis l'autre chambre dans la chambre (22) au moyen de la compression de l'autre chambre.

8. Procédé de fabrication d'un dispositif d'administration (1) en particulier selon l'une des revendications précédentes, comprenant :
la préparation d'une première section de film (21) fabriquée dans un processus d'emboutissage profond ;
la formation d'une chambre (22) dans la première section de film (21) ;
la préparation d'une seconde section de film (23) fabriquée de préférence dans un processus d'emboutissage profond et fabriquée de préférence à partir d'un film aluminium et conçue de préférence de manière plane ;
la stérilisation de la première section de film (21) et de la seconde section de film (23) ;
le positionnement d'un adaptateur de bec verseur (3) comportant un raccord ;
le remplissage de la chambre (22) d'une substance fluide et stérile à administrer dans des conditions stériles ;
la création d'une liaison (4) de la première section de film (21) avec la seconde section de film (23) dans des conditions stériles, de sorte que
l'adaptateur de bec verseur (3) est relié de manière étanche au corps de base, et
la liaison (4) présente plusieurs zones qui comprennent une zone plus solide (41) et une zone moins solide (42) agencée entre la chambre (22) et la chambre de sortie de l'ouverture de sortie (24), dans lequel
la zone moins solide (42) de la liaison (4) est conçue pour se détacher lors de la compression de la chambre (22) pour l'évacuation de la substance à travers la chambre de sortie de l'ouverture de sortie (24), et
la zone plus solide (41) de la liaison (4) est conçue pour rester reliée lors de la compression de la chambre (22) pour l'évacuation de la substance à travers la chambre de sortie de l'ouverture de sortie (24),
de sorte que la substance, au moyen de la compression de la chambre (22), peut être évacuée à travers la chambre de sortie de l'ouverture de sortie (24) quasiment exclusivement à travers l'ouverture de sortie (24) et à travers le raccord (31) de l'adaptateur de bec verseur (3), dans lequel
une pièce adaptée, qui est adaptée au mode d'administration souhaité de la substance, comme la projection, goutte-à-goutte, application ou injection, peut être montée sur le raccord de l'adaptateur de bec verseur, dans lequel la pièce comprend un bec verseur, un distributeur de gouttelettes ou une aiguille d'injection d'une longueur souhaitée, et dans lequel l'adaptateur de bec verseur (3) présente une section de scellement (32) qui possède une forme quasiment triangulaire en vue de face, et dans lequel
la section de scellement (32) est agencée entre le premier film (21) et le second film (23) et est reliée de manière fixe à ceux-ci.

9. Procédé selon la revendication 8, dans lequel l'adaptateur de bec verseur (3)
est fabriqué à partir d'un matériau solide qui, lors d'une compression de la chambre (22) pour l'évacuation de la substance hors de la chambre (22), retient sensiblement sa forme, et/ou
lors de la fermeture de la chambre (22) par une liaison fixe de la première section de film (21) avec la seconde section de film (23), est agencé entre la première section de film (21) et la seconde section de film (23) et est relié à celles-ci de manière étanche, dans lequel l'adaptateur de bec verseur (3) présente de préférence une section de scellement qui est agencée entre la première section de film (21) et la seconde section de film (23) et reliée de manière fixe à celles-ci, et/ou
lors du positionnement et avant le remplissage de la chambre, est fixé à la première section de film.

10. Procédé selon l'une des revendications 8 à 9, dans lequel la première section de film (21) est constituée d'une matière plastique fabriquée dans un processus d'emboutissage profond ou d'un aluminium fabriqué dans un processus d'emboutissage profond.

11. Procédé selon l'une des revendications 8 à 10, dans lequel
la première section de film (21) est conçue comme un premier film (21) et la seconde section de film (23) est conçue comme un second film (23), dans lequel le premier film (21) est relié de manière fixe au second film (23), et/ou
la première section de film (21) ou la seconde section de film (23) ou la première section de film (21) et la seconde section de film (23) sont pourvues d'une couche antiseptique.

12. Procédé selon l'une des revendications 8 à 11, dans lequel la liaison (4) de la première section de film (21) à la seconde section de film (23) est créée dans des conditions stériles par scellement thermique.

13. Procédé selon l'une des revendications 8 à 12, dans lequel le raccord (31) de l'adaptateur de bec verseur (3) est conforme à une norme ou est réalisé comme un raccord Luer-Lock (31).

14. Procédé selon l'une des revendications 8 à 13, dans lequel la liaison (4) est créée par des joints scellés, dans lequel de préférence les joints scellés de la liaison (4) sont fabriqués par scellement thermique et/ou la zone plus solide (41) de la liaison (4) est créée par des joints scellés comportant une plus grande résistance et la zone moins solide (42) de la liaison (4) est créée par des joints scellés comportant une plus faible résistance.
